(19) Europäisches Patentamt
European Patent Office
Office européen des brevets

(11) **EP 4 425 395 A1**

(12) **EUROPEAN PATENT APPLICATION**
published in accordance with Art. 153(4) EPC

(43) Date of publication:
**04.09.2024 Bulletin 2024/36**

(21) Application number: **22885322.2**

(22) Date of filing: **15.08.2022**

(51) International Patent Classification (IPC):
**G06Q 10/06** $^{(2023.01)}$     **G16C 20/10** $^{(2019.01)}$

(52) Cooperative Patent Classification (CPC):
Y02P 90/845

(86) International application number:
**PCT/CN2022/112575**

(87) International publication number:
**WO 2023/071422 (04.05.2023 Gazette 2023/18)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.10.2021 CN 202111267061**

(71) Applicants:
• **Guangdong Brunp Recycling Technology Co., Ltd.**
  **Foshan, Guangdong 528137 (CN)**
• **Hunan Brunp Recycling Technology Co., Ltd.**
  **Changsha, Hunan 410600 (CN)**

• **Hunan Brunp EV Recycling Co., Ltd.**
  **Changsha, Hunan 410600 (CN)**

(72) Inventors:
• **YU, Haijun**
  **Foshan, Guangdong 528137 (CN)**
• **XIE, Yinghao**
  **Foshan, Guangdong 528137 (CN)**
• **LI, Aixia**
  **Foshan, Guangdong 528137 (CN)**
• **ZHANG, Xuemei**
  **Foshan, Guangdong 528137 (CN)**
• **LI, Changdong**
  **Foshan, Guangdong 528137 (CN)**

(74) Representative: **Germain Maureau**
  **12, rue Boileau**
  **69006 Lyon (FR)**

(54) **ACCOUNTING SYSTEM FOR CARBON EMISSIONS OF LITHIUM BATTERY POSITIVE ELECTRODE MATERIAL SINTERING**

(57)     The present invention relates to the technical field of carbon emission accounting, and discloses an carbon emission accounting system for sintering of lithium battery cathode materials, including: a material balance module, an energy balance module, a carbon emission accounting module, a data module, and a knowledge module, wherein the material balance module is to read a formula and data to account a loss of lithium during the sintering process; the energy balance module is to read a data to account an energy loss during the sintering process; the carbon emission accounting module is to read a formula, data, the loss of lithium element, and the energy loss to account carbon emission during the sintering process; the data module is used to store data; and the knowledge module is used to store formulas and parameter values in the accounting process. The present invention fully considers carbon emission involved in the entire sintering process of cathode material by adopting a material balance module, an energy balance module, and a carbon emission accounting module, which can quickly and efficiently account overall carbon emission through inputted data and pre-stored accounting formulas.

FIG. 1

**Description**

**TECHNICAL FIELD**

The present invention relates to the technical field of carbon emission accounting, in particular to a carbon emission accounting system for sintering of lithium battery cathode materials.

**BACKGROUND**

At present, with the goal of carbon neutrality, the power system is undergoing transformation. In a newly formed power system, an energy storage system with a main part consisting of traction batteries needs to be deeply involved in the transformation. However, with the rapid growth of scale and volume of production capacity for traction batteries, a large amount of carbon emissions will be generated in the production process of traction batteries as well as the upstream and downstream industrial chain. Moreover, traction batteries plays important part in the carbon emissions of the entire life cycle of electric vehicles, and the carbon emissions of traction batteries have become a core concern in the transportation field.

The carbon emissions of traction batteries cannot be underestimated, and are mainly concentrated in two processes, where one of them is the production of battery cells, and the other is the production of key materials such as upstream positive electrodes and negative electrodes. Furthermore, in the production of key materials for lithium batteries, the production of cathode materials consumes the most energy. In order to achieve the goal of carbon neutrality, an accounting system for carbon emissions from the sintering of lithium battery cathode materials is needed to account the carbon emissions during the preparation process of lithium battery cathode materials.

**SUMMARY**

The purpose of the present invention is to provide a carbon emission accounting system for sintering of a lithium battery cathode material to account carbon emission during preparation of lithium battery cathode material.

In order to achieve the above objective, the present invention provides a carbon emission accounting system for sintering of lithium battery cathode materials, including: a material balance module, an energy balance module, a carbon emission accounting module, a data module, and a knowledge module, where the material balance module is used to read a first formula from the knowledge module and a first data from the data module to account loss of lithium element during the sintering process of the lithium battery cathode material; the energy balance module is used to read a second formula from the knowledge module and a second data from the data module to account an energy loss during the sintering process of the lithium battery cathode material; the carbon emission accounting module is used to read a third formula from the knowledge module, and a third data, the loss of lithium element and the energy loss from the data module to account carbon emissions during the sintering process of the lithium battery cathode material; the data module is used to store the first data, the second data, the third data and an intermediate data generated in the accounting system; and the knowledge module is used to store the first formula, the second formula, the third formula and parameter values in the accounting process.

Further, the first data includes: a mass of a loaded sagger before sintering, a mass of crystal water in a precursor, a mass of a sagger discharged after sintering, a molar amount of the precursor in a raw material before sintering, and a molar amount of lithium carbonate in the raw material before sintering; and the first formula is specifically:

$$M_{Li\,loss} = M_{before} - M_{water} - M_{after} - 10a - 44b;$$

where, $M_{Li\,loss}$ represents the loss of lithium, $M_{before}$ represents the mass of a loaded sagger before sintering, $M_{water}$ represents the mass of crystal water in the precursor, $M_{after}$ represents the mass of the sagger discharged after sintering, and a represents the molar amount of the precursor in the raw material before sintering; and b represents the molar amount of lithium carbonate in the raw material before sintering.

Further, the second data includes: electrical energy consumed in mixing, transportation, sintering, crushing, sieving, iron removal, and packaging of materials; and the second formula is specifically:

$$E_{total} = \sum_{i=1}^{N} E_i$$

where, $E_{total}$ represents total electrical energy consumed, and $E_i$ represents the electrical energy consumed in mixing, transportation, sintering, crushing, sieving, iron removal, and packaging processes of the materials, respectively.

Further, the third data includes: types and quantities of raw materials, water vapor directly discharged from the sintering process, carbon dioxide directly discharged from the sintering process, and carbon emission conversion coefficients corresponding to different substances.

The third formula is specifically:

$$C_{total} = C_{yc} + C_{electricity} + C_{water} + C_{carbon} + C_{Li\ loss}$$

where, $C_{total}$ represents the total carbon emission, $C_{yc}$ represents carbon emission from raw material manufacturing, $C_{electricity}$ represents carbon emission converted from total electrical energy consumed, and $C_{water}$ represents carbon emission converted from water vapor directly discharged from the sintering process; $C_{carbon}$ represents the carbon dioxide directly emitted during the sintering process, and $C_{Li\ loss}$ represents carbon emission converted from the lithium loss.

Further, the carbon emission converted from the total electric energy consumed is specifically:
carbon emission caused by the consumed electric energy, as calculated based on a global warming potential value of greenhouse gases.

Further, the accounting system further includes a data input module; and the data input module is used to input the first data, the second data, the third data, the first formula, the second formula, and the third formula.

Further, the accounting system further includes a data output and display module; and the data output and display module is used to output and display the accounting result.

Further, the accounting system further includes an energy loss accounting module; and the energy loss accounting module is used to read a fourth formula from the knowledge module and a fourth data from the data module to account an energy loss during the sintering process.

Further, the fourth data includes: start time for sintering, end time for sintering, a heat exchange coefficient between a kiln and external environment, temperature of the roller kiln, temperature of the external environment, and total electric energy consumed by the roller kiln during the sintering process.

The fourth formula is specifically:

$$E_{loss} = \int_{t1}^{t2} \varepsilon \cdot (T_1 - T_2) \cdot E_{klin} dt$$

where, $E_{loss}$ represents the energy lost during the sintering process; t1 represents the start time for sintering; t2 represents the end time for sintering; $\varepsilon$ represents the heat transfer coefficient between the kiln and the external environment; $T_1$ represents the temperature of the roller kiln; $T_2$ represents the external ambient temperature; and $E_{kiln}$ represents the total electric energy consumed by the roller kiln during the sintering process.

Further, the accounting system includes an analysis module that is used to obtain an energy loss calculated by the energy loss accounting module and loss of lithium element calculated by the material balance module, establish an analysis model based on the energy loss and the loss of lithium element, and determine a correlation coefficient between the energy loss and the loss of lithium element.

In comparison with prior art, a carbon emission accounting system for sintering of a lithium battery cathode materials of the present invention has the following beneficial effect: the present invention fully considers the carbon emissions involved in an entire sintering process of cathode material by adopting a material balance module, an energy balance module, and a carbon emission accounting module. A total carbon emission can be accounted quickly and efficiently through inputted data and pre-stored calculation formulas.

**BRIEF DESCRIPTION OF DRAWINGS**

FIG 1 is a schematic diagram of the overall structure of a carbon emission accounting system for sintering of a lithium battery cathode material according to the present invention;
FIG. 2 is a schematic diagram of sintering process of a lithium battery cathode material according to the present invention.

**DETAILED DESCRIPTION**

The specific implementation of the present invention will be described in further detail below in conjunction with the accompanying drawings and embodiment examples. The following embodiment examples are used to illustrate the present invention, but not to limit the scope of the present invention.

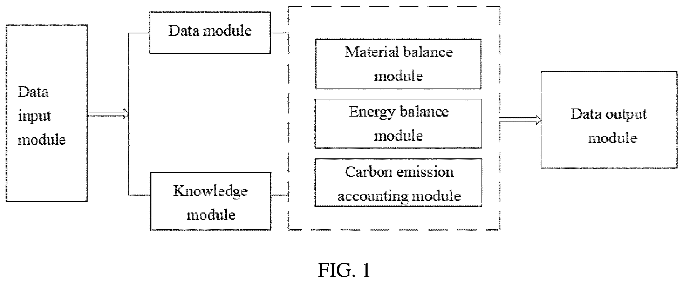

As shown in Figure 1, the present invention discloses an carbon emission accounting system for sintering process of lithium battery cathode material, including: a material balance module, an energy balance module, a carbon emission accounting module, a data module, and a knowledge module; the material balance module is used to read a first formula from the knowledge module, and a first data from the data module to account loss of lithium element during the sintering of the lithium battery cathode material; the energy balance module is used to read a second formula from the knowledge module and a second data from the data module to account an energy loss during the sintering of the lithium battery cathode material; the carbon emission accounting module is used to read a third formula from the knowledge module, and a third data, the loss of lithium element and the energy loss from the data module to account a carbon emission in the sintering of the lithium battery cathode material; the data module is used to store the first data, the second data, the third data and an intermediate data generated in the accounting system; the knowledge module is used to store the first formula, the second formula, the third formula and parameter values in the accounting process.

In the present invention, the carbon emissions involved in the entire sintering process of cathode material is fully considered by means of the material balance module, the energy balance module and the carbon emission accounting module. Through an input data and pre-stored accounting formulas, the accounting can be carried out quickly and efficiently to obtain overall carbon emission.

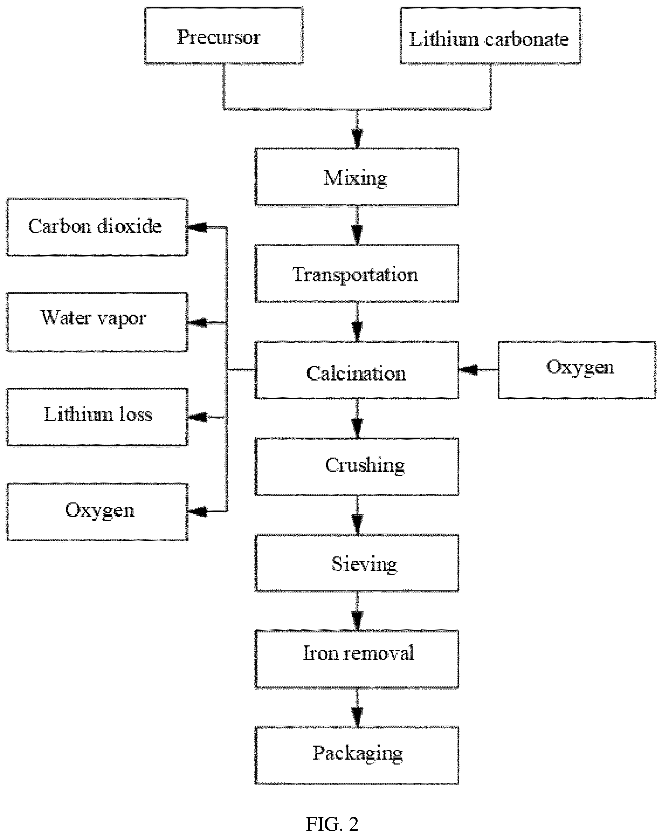

In order to better describe the technical solution of the present invention, referring to FIG. 2, taking the sintering of an NCM523 type cathode material as an example, which involves a nickel cobalt manganese oxide having a chemical formula of $Ni_{0.5}Co_{0.2}Mn_{0.3}(OH)_2$ and in a Ni: Co: Mn molar ratio of 5: 2: 3, and lithium carbonate as a lithium source.

In this embodiment example, the first data includes: a mass of a loaded sagger before sintering, a mass of crystal water in the precursor, a mass of a sagger discharged after sintering, a molar amount of the precursor in the raw material before sintering, and a molar amount of lithium carbonate in the raw material before sintering; and the first formula is specifically:

$$M_{Li\,loss} = M_{before} - M_{water} - M_{after} - 10a - 44b;$$

where, $M_{Li\,loss}$ represents the loss of lithium, $M_{before}$ represents the mass of the loaded sagger before sintering, $M_{water}$ represents the mass of crystal water in the precursor, $M_{after}$ represents the mass of the sagger discharged after sintering, and a represents the molar amount of the precursor in the raw material before sintering; and b represents the molar amount of lithium carbonate in the raw material before sintering.

In this embodiment example, the first data includes: the mass of the loaded saggar before sintering, the mass of crystal water in the precursor $(Ni_{0.5}Co_{0.2}Mn_{0.3}(OH)_2)$, and a saggar mass after sintering, the molar amount of precursor $(Ni_{0.5}Co_{0.2}Mn_{0.3}(OH)_2)$ in a raw material before sintering and a molar amount of lithium carbonate in a raw material before sintering.

In this embodiment example, the material change in the sintering process are as follows: at 100-200°C, the precursor decrystallizes water; at 400°C, the precursor hydroxide is dehydrated to form oxide; at 500°C, lithium carbonate is decomposed to form lithium oxide; and at 700°C, the precursor oxide reacts with lithium oxide to form lithium nickel cobalt manganese oxide.

a mol of nickel cobalt manganese hydroxide precursor and b mol of lithium carbonate are weighed and mixed. Sintering is performed to a resulting mixture in an oxygen atmosphere. The precursor and lithium carbonate will react in a ratio of 1: 1 to form a lithium nickel cobalt manganese oxide cathode material. Part of an excess lithium remains on the surface of cathode material in a form of lithium oxide, and the other part enters the external environment with airflow in a form of lithium oxide, thereby causing carbon emissions. The reaction equation is as follows:

$$O_2+4Me(OH)_2+2Li_2CO_3==4LiMeO_2+4H_2O+2CO_2;$$

Me is Ni, Co, Mn. $M_{before}$ represents the sagger mass before sintering, which is a total mass of the sagger and the mixture, and $M_{after}$ represents the sagger mass after sintering, which is a total mass of the sagger, lithium nickel cobalt manganese oxide, and residual lithium.

During the sintering process, the mass of water discharged is the mass of crystal water plus the mass of water produced by the reaction, that is, $M_{water}$ + a mol water, carbon dioxide is b mol, and oxygen participating in the reaction is a/4 mol. Therefore, the lithium loss mass (accounted as LizO) is $M_{before}$-$M_{after}$-$M_{water}$-a mol water-b mol carbon dioxide + a/4 mol oxygen, and the following formula is obtained:

$$M_{Li\ loss} = M_{before} - M_{water} - M_{after} - 10a - 44b$$

In this embodiment example, a second data includes: electrical energy consumed in the mixing, transportation, sintering, crushing, sieving, iron removal, and packaging of materials; and the second formula is specifically:

$$E_{total} = \sum_{i=1}^{N} E_i$$

where, $E_{total}$ represents total electrical energy consumed, and $E_i$ represents the electrical energy consumed in the mixing, transportation, sintering, crushing, sieving, iron removal, and packaging processes of the materials respectively.

In this embodiment example, the third data includes: types and quantities of raw materials, water vapor directly discharged from the sintering process, carbon dioxide directly discharged from the sintering process, and carbon emission conversion coefficients corresponding to different substances;

The third formula is specifically:

$$C_{total} = C_{yc} + C_{electricity} + C_{water} + C_{carbon} + C_{Li\ loss}$$

where, $C_{total}$ represents the total carbon emission, $C_{yc}$ represents carbon emission from raw material manufacturing, $C_{electricity}$ represents carbon emission converted from the total electrical energy consumed, and $C_{water}$ represents carbon emission converted from water vapor directly discharged from the sintering process; $C_{carbon}$ represents the carbon dioxide directly emitted during the sintering process, and $C_{Li\ loss}$ represents carbon emission converted from the lithium loss.

In this embodiment example, the raw materials include precursors, lithium carbonate, and oxygen.

In this embodiment example, the amount of lithium loss can be measured by the molar mass of the precursor and lithium carbonate (which can be obtained by weighing before mixing) and the mass of the loaded sagger before and after sintering. The oxygen consumption can be obtained through an oxygen flowmeter. By looking up the carbon emission factor, you can convert the carbon emissions from the precursor, lithium carbonate, oxygen and other raw materials, as well as the total electricity energy consumption, water vapor emission, carbon dioxide emission, lithium loss emission, etc.; and finally get a total carbon emission.

In this embodiment example, the carbon emission converted from the total electric energy consumed is specifically:

A carbon emission caused by the consumed electric energy, as calculated based on a global warming potential value of greenhouse gases.

In this embodiment example, the accounting system further includes a data input module; the data input module is used to input the first data, the second data, the third data, the first formula, the second formula, and the third formula.

In this embodiment example, the data inputted by the data input module includes: x kg of the precursor with a water content of 0.5% which is accurately weighed, that is, a mol $Ni_{0.5}Co_{0.2}Mn_{0.3}(OH)_2$ and 0.005x kg of crystal water; y kg of lithium carbonate, that is, b mol of lithium carbonate; electrical energy E1 consumed by mixing, electrical energy E2 consumed by transportation, electrical energy E3 consumed by sintering..., which are derived from electric meters for input; the mass of the filled sagger before sintering denoted as M1; the mass of the sagger discharged after sintering denoted as M2; oxygen used in the sintering process derived from a flowmeter for input; the carbon emission factor of $Ni_{0.5}Co_{0.2}Mn_{0.3}(OH)_2$ with 0.5% water content; the carbon emission factor of lithium carbonate, the carbon emission factor of oxygen, the carbon emission factor of electric energy conversion, the carbon emission factor of water vapor, and the carbon emission factor of lithium oxide.

In this embodiment example, the accounting system further includes a data output and display module; and the data output and display module is used to output and display the accounting result.

In this embodiment example, the output and display content includes:

$$M_{Li\ loss} = M_1 - 0.005x - M_2 - 10a - 44b$$

$$E_{total} = E1 + E2 + E3 + ...$$

$$C_{total} = C_{yc} + C_{electricity} + C_{water} + C_{carbon} + C_{Li\,loss}$$

$$E_{loss} = \int_{t1}^{t2} \varepsilon \cdot (T_1 - T_2) \cdot E_{klin} dt$$

In this embodiment example, the accounting system further includes an energy loss accounting module; the energy loss accounting module is used to read a fourth formula from the knowledge module and a fourth data from the data module to account the energy loss during the sintering process.

In this embodiment example, the fourth data includes: start time for sintering, end time for sintering, a heat exchange coefficient between a kiln and external environment, temperature of the roller kiln, temperature of the external environment, and total electric energy consumed by the roller kiln during the sintering process.

The fourth formula is specifically:

$$E_{loss} = \int_{t1}^{t2} \varepsilon \cdot (T_1 - T_2) \cdot E_{klin} dt$$

Among them, $E_{loss}$ represents the energy lost during the sintering process; t1 represents the start time for sintering; t2 represents the end time for sintering; $\varepsilon$ represents the heat transfer coefficient between the kiln and the external environment; $T_1$ represents the temperature of the roller kiln; $T_2$ represents the external ambient temperature; and $E_{kiln}$ represents the total electric energy consumed by the roller kiln during the sintering process.

In this embodiment example, the accounting system further includes an analysis module that is used to obtain an energy loss calculated by the energy loss accounting module and loss of lithium element calculated by the material balance module, establish an analysis model based on the energy loss and the loss of lithium element, and determine a correlation coefficient between the energy loss and the loss of lithium element.

To sum up, compared with the prior art, a system for calculating carbon emissions from sintering of lithium battery cathode materials in an embodiment example of the present invention has the following beneficial effect: the present invention fully considers the carbon emissions involved in the entire sintering process of cathode material by adopting a material balance module, an energy balance module, and a carbon emission accounting module. A total carbon emission can be accounted quickly and efficiently through inputted data and pre-stored calculation formulas.

The invention additionally adds an accounting module for the energy loss in the sintering process, to study the influence of the roller kiln temperature change during a heating stage and a heat preservation stage on the lithium loss, thereby exploring an optimal sintering process for further reducing lithium loss and decreasing carbon emissions.

The above are only the preferred embodiment examples of the present invention. It should be pointed out that, for those ordinary skilled in the art, several improvements and substitutions can be made without departing from the technical principles of the present invention. These improvements and substitutions should also be regarded as within the protection scope of the present invention.

**Claims**

1. A carbon emission accounting system for sintering of a lithium battery cathode material, comprising: a material balance module, an energy balance module, a carbon emission accounting module, a data module, and a knowledge module, wherein the material balance module is used to read a first formula from the knowledge module and a first data from the data module to account loss of lithium element during the sintering process of the lithium battery cathode material; the energy balance module is used to read a second formula from the knowledge module and a second data from the data module to account an energy loss during the sintering process of the lithium battery cathode material; the carbon emission accounting module is used to read a third formula from the knowledge module, and a third data, the loss of lithium element and the energy loss from the data module to account carbon emission during the sintering process of the lithium battery cathode material; the data module is used to store the first data, the second data, the third data and an intermediate data generated in the accounting system; and the knowledge module is used to store the first formula, the second formula, the third formula and parameter values in the accounting process.

2. The carbon emission accounting system for sintering of the lithium battery cathode material according to claim 1, wherein the first data includes: a mass of a loaded sagger before sintering, a mass of crystal water in a precursor, a mass of a sagger discharged after sintering, a molar amount of the precursor in the raw material before sintering, and a molar amount of lithium carbonate in the raw material before sintering; and the first formula is specifically:

$$M_{Li\ loss} = M_{before} - M_{water} - M_{after} - 10a - 44b;$$

where, $M_{Li\ loss}$ represents the loss of lithium, $M_{before}$ represents the mass of the loaded sagger before sintering, $M_{water}$ represents the mass of crystal water in the precursor, $M_{after}$ represents the mass of the sagger discharged after sintering, and a represents the molar amount of the precursor in the raw material before sintering; and b represents the molar amount of lithium carbonate in the raw material before sintering.

3. The carbon emission accounting system for sintering of the lithium battery cathode material according to claim 1, wherein the second data includes: electrical energy consumed in mixing, transportation, sintering, crushing, sieving, iron removal, and packaging of materials; and the second formula is specifically:

$$E_{total} = \sum_{i=1}^{N} E_i$$

where, $E_{total}$ represents total electrical energy consumed, and $E_i$ represents the electrical energy consumed in mixing, transportation, sintering, crushing, sieving, iron removal, and packaging processes of the materials respectively.

4. The carbon emission accounting system for sintering of the lithium battery cathode material according to claim 1, wherein the third data includes: type and quantity of raw materials, water vapor directly discharged from the sintering process, carbon dioxide directly discharged from the sintering process, and carbon emission conversion coefficients corresponding to different substances; and
the third formula is specifically:

$$C_{total} = C_{yc} + C_{electricity} + C_{water} + C_{carbon} + C_{Li\ loss}$$

where, $C_{total}$ represents the total carbon emission, $C_{yc}$ represents carbon emission from raw material manufacturing, $C_{electricity}$ represents carbon emission converted from total electrical energy consumed, and $C_{water}$ represents carbon emission converted from the water vapor directly discharged from the sintering process; $C_{carbon}$ represents carbon dioxide directly emitted during the sintering process, and $C_{Li\ loss}$ represents carbon emission converted from the lithium loss.

5. The carbon emission accounting system for sintering of the lithium battery cathode material according to claim 4, wherein the carbon emission converted from the total electric energy consumed is specifically:
carbon emission caused by the consumed electric energy, as calculated based on a global warming potential value of greenhouse gases.

6. The carbon emission accounting system for sintering of the lithium battery cathode material according to claim 1, wherein the accounting system further includes a data input module; and the data input module is used to input the first data, the second data, the third data, the first formula, the second formula, and the third formula.

7. The carbon emission accounting system for sintering of the lithium battery cathode material according to claim 1, wherein the accounting system further includes a data output and display module; and the data output and display module is used to output and display an accounting result.

8. The carbon emission accounting system for sintering of the lithium battery cathode material according to claim 1, wherein the accounting system further includes an energy loss accounting module; and the energy loss accounting module is used to read a fourth formula from the knowledge module and a fourth data from the data module to account an energy loss during the sintering process.

**9.** The carbon emission accounting system for sintering of the lithium battery cathode material according to claim 8, wherein the fourth data includes: start time for sintering, end time for sintering, a heat exchange coefficient between a kiln and external environment, temperature of the roller kiln, temperature of the external environment, and total electric energy consumed by the roller kiln during the sintering process; and

the fourth formula is specifically:

$$E_{loss} = \int_{t1}^{t2} \varepsilon \cdot (T_1 - T_2) \cdot E_{klin} dt$$

where, $E_{loss}$ represents the energy lost during the sintering process; t1 represents the start time for sintering; t2 represents the end time for sintering; $\varepsilon$ represents the heat transfer coefficient between the kiln and the external environment; $T_1$ represents the temperature of the roller kiln; $T_2$ represents the external ambient temperature; and $E_{kiln}$ represents the total electric energy consumed by the roller kiln during the sintering process.

**10.** The carbon emission accounting system for sintering of the lithium battery cathode material according to claim 8, wherein the accounting system further includes an analysis module that is used to obtain an energy loss calculated by the energy loss accounting module and loss of lithium element calculated by the material balance module, establish an analysis model based on the energy loss and the loss of lithium element, and determine a correlation coefficient between the energy loss and the loss of lithium element.

FIG. 1

FIG. 2

## INTERNATIONAL SEARCH REPORT

| International application No. |
| --- |
| **PCT/CN2022/112575** |

### A. CLASSIFICATION OF SUBJECT MATTER

G06Q 10/06(2012.01)i; G16C 20/10(2019.01)n

According to International Patent Classification (IPC) or to both national classification and IPC

### B. FIELDS SEARCHED

Minimum documentation searched (classification system followed by classification symbols)

G06Q; G16C

Documentation searched other than minimum documentation to the extent that such documents are included in the fields searched

Electronic data base consulted during the international search (name of data base and, where practicable, search terms used)

CNTXT; WPABSC; ENTXTC; WPABS; ENTXT; CJFD; CNKI; Bing: 电池, 锂, 正极, 负极, 烧结, 烧制, 焙烧, 煅烧, 生产, 制备, 制造, 碳排放, 碳足迹, 核算, 计算, 评价, 分析, 守恒, 平衡, 能量, 电能, battery, li, cell, positive, electrode, sinter, fir, calcinate, preparate, production, co2, carbon, emission, footprint, balance, energy, power

### C. DOCUMENTS CONSIDERED TO BE RELEVANT

| Category* | Citation of document, with indication, where appropriate, of the relevant passages | Relevant to claim No. |
| --- | --- | --- |
| PX | CN 114067922 A (GUANGDONG BRUNP RECYCLING TECHNOLOGY CO., LTD. et al.) 18 February 2022 (2022-02-18) claims 1-10 | 1-10 |
| X | 张铭 (ZHANG, Ming). "稀土磁性材料企业碳排放核算与分析 (Carbon Emission Accounting and Analysis of Rare Earth Magnetic Material Enterprises)" 中国优秀硕士学位论文全文数据库工程科技I辑 (Chinese Master's Theses Full-text Database, Engineering Science & Technology I), No. 01, 15 January 2021 (2021-01-15), ISSN: 1674-0246, Abstract, chapter 4 | 1-10 |
| X | CN 106709265 A (UNIVERSITY OF SHANGHAI FOR SCIENCE AND TECHNOLOGY) 24 May 2017 (2017-05-24) description, paragraphs [0068]]-0153] | 1-10 |
| A | CN 106467929 A (ZHANG GUICHUN) 01 March 2017 (2017-03-01) entire document | 1-10 |
| A | CN 113516371 A (HANGZHOU HUIYUAN ZHIGU TECHNOLOGY CO., LTD. et al.) 19 October 2021 (2021-10-19) entire document | 1-10 |

☑ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **12 October 2022** | **27 October 2022** |

| Name and mailing address of the ISA/CN | Authorized officer |
| --- | --- |
| **China National Intellectual Property Administration (ISA/ CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.

**PCT/CN2022/112575**

| C. | DOCUMENTS CONSIDERED TO BE RELEVANT | |
|---|---|---|
| Category* | Citation of document, with indication, where appropriate, of the relevant passages | Relevant to claim No. |
| A | US 2012053748 A1 (BRANSCOMB BENNETT HILL) 01 March 2012 (2012-03-01) entire document | 1-10 |

Form PCT/ISA/210 (second sheet) (January 2015)

**INTERNATIONAL SEARCH REPORT**
Information on patent family members

International application No.

**PCT/CN2022/112575**

| Patent document cited in search report | | | Publication date (day/month/year) | Patent family member(s) | | | Publication date (day/month/year) |
|---|---|---|---|---|---|---|---|
| CN | 114067922 | A | 18 February 2022 | None | | | |
| CN | 106709265 | A | 24 May 2017 | None | | | |
| CN | 106467929 | A | 01 March 2017 | None | | | |
| CN | 113516371 | A | 19 October 2021 | None | | | |
| US | 2012053748 | A1 | 01 March 2012 | US | 9385531 | B2 | 05 July 2016 |

Form PCT/ISA/210 (patent family annex) (January 2015)